(19) Europäisches Patentamt
European Patent Office
Office européen des brevets

(11) Veröffentlichungsnummer: **0 450 743 A1**

(12) **EUROPÄISCHE PATENTANMELDUNG**

(21) Anmeldenummer: **91250091.5**

(51) Int. Cl.5: **C07C 401/00**

(22) Anmeldetag: **08.04.91**

(30) Priorität: **06.04.90 DE 4011682**

(43) Veröffentlichungstag der Anmeldung:
**09.10.91 Patentblatt 91/41**

(84) Benannte Vertragsstaaten:
**AT BE CH DE DK ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**W-1000 Berlin 65(DE)**

(72) Erfinder: **Neef, Günter, Dr.**
**Markgraf-Albrecht-Str. 4**
**W-1000 Berlin 31(DE)**
Erfinder: **Kirsch, Gerald, Dr.**
**Luciusstr. 6b**
**W-1000 Berlin 33(DE)**
Erfinder: **Steinmeyer, Andreas, Dr.**
**Karolingerplatz 3**
**W-1000 Berlin 19(DE)**
Erfinder: **Schwarz, Katica**
**Zillestrasse 109**
**W-1000 Berlin 10(DE)**
Erfinder: **Bräutigam, Matthias, Dr.**
**Droysenstrasse 8**
**W-1000 Berlin 12(DE)**
Erfinder: **Thieroff-Ekerdt, Rith, Dr.**
**Am Vierrutenberg**
**W-1000 Berlin 28(DE)**
Erfinder: **Rach, Petra**
**Antwerpener Str. 1**
**W-1000 Berlin 65(DE)**

(54) **24-Oxa-Derivate in der Vitamin D-Reihe.**

(57) Es werden neue 24-Oxa-Derivate in der Vitamin D-Reihe der allgemeinen Formel I

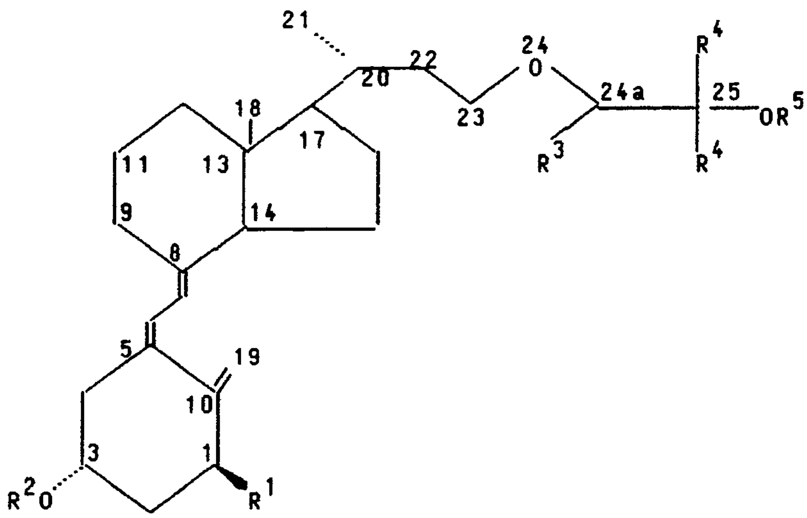

(I),

beschrieben, worin

$R^1$, $R^2$ und $R^5$ unabhängig voneinander ein Wasserstoffatom oder eine Acylgruppe mit 1 bis 9 Kohlenstoff-

EP 0 450 743 A1

atomen

$R^3$ ein Wasserstoffatom oder eine lineare oder verzweigte Alkylgruppe mit 1 bis 4 Kohlenstoffatomen und

$R^4$ je ein Wasserstoffatom oder je eine lineare oder verzweigte Alkylgruppe mit 1 bis 4 Kohlenstoffatomen

bedeuten, ein Verfahren zu deren Herstellung, pharmazeutische Präparate, die diese Verbindungen enthalten sowie deren Verwendung zur Herstellung von Arzneimitteln.

Die vorliegende Erfindung betrifft 24-Oxa-Derivate in der Vitamin D-Reihe der allgemeinen Formel I

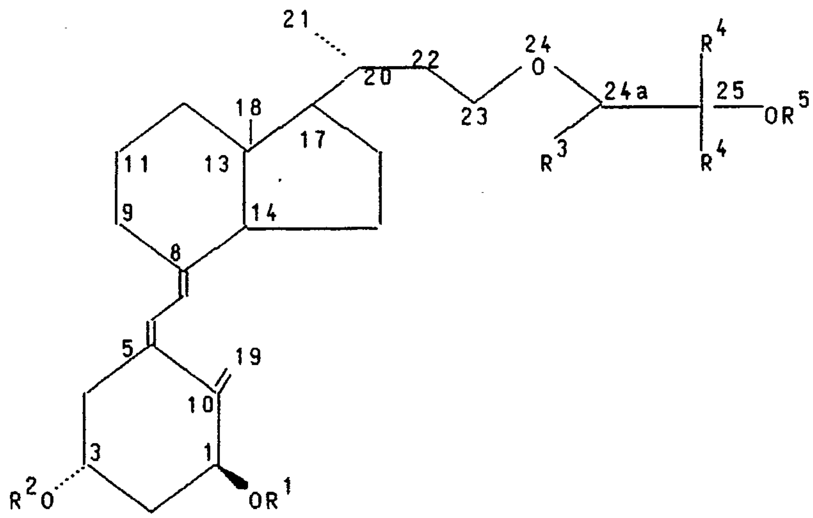

(I),

worin

| | |
|---|---|
| $R^1$, $R^2$ und $R^5$ | unabhängig voneinander ein Wasserstoffatom oder eine Acylgruppe mit 1 bis 9 Kohlenstoffatomen, |
| $R^3$ | ein Wasserstoffatom oder eine lineare oder verzweigte Alkylgruppe mit 1 bis 4 Kohlenstoffatomen und |
| $R^4$ | je ein Wasserstoffatom oder je eine lineare oder verzweigte Alkylgruppe mit 1 bis 4 Kohlenstoffatomen |

bedeuten,

ein Verfahren zu deren Herstellung, pharmazeutische Präparate, die diese Verbindungen enthalten sowie deren Verwendung zur Herstellung von Arzneimitteln. Die für die Reste $R^1$, $R^2$ und $R^5$ möglichen Acylgruppen mit 1 bis 9 Kohlenstoffatomen sind insbesondere von gesättigten Carbonsäuren oder auch von der Benzoesäure abgeleitet. Andere geeignete Acylreste $R^1$, $R^2$, $R^5$ umfassen solche, die cyclisch, acyclisch, carbocyclisch oder heterocyclisch - alle gegebenenfalls auch ungesättigt - sind. Die bevorzugten Reste leiten sich von $C_1$ - bis $C_9$, insbesondere $C_2$ - bis $C_5$ -, Alkancarbonsäuren ab, wie beispielsweise Acetyl-, Propionyl-, Butyryl-.

Bei den Alkylgruppen $R^3$ und $R^4$, die geradkettig oder verzweigt sein können, kommen in erster Linie die Methyl-, Ethyl-, Propyl- und für $R^3$ zusätzlich die Isopropylgruppe und für $R^4$ zusätzlich die n-Butylgruppe inbetracht. $R^3$ und $R^4$ können identisch oder unterschiedlich sein.

Besonders bevorzugt sind gemäß vorliegender Erfindung die folgenden Verbindungen:

1α,25-Dihydroxy-24-oxa-24-homo-cholecalciferol
1a,25-Dihydroxy-26,27-dimethyl-24-oxa-24-homo-cholecalciferol
26,27-Diethyl-1α,25-dihydroxy-24-oxa-24-homo-cholecalciferol
1α,25-Dihydroxy-24-oxa-26,27-di-n-propyl-24-homo-cholecalciferol
1α,26-Dihydroxy-24-oxa-cholecalciferol
1α,26-Dihydroxy-27-methyl-24-oxa-cholecalciferol

Die natürlichen Vitamine $D_2$ und $D_3$ (vgl. allgemeine Formel A) sind an sich biologisch inaktiv und werden erst nach Hydroxylierung in 25-Position in der Leber bzw. in 1-Position in der Niere in deren biologisch aktive Metaboliten umgewandelt. Die Wirkung der Vitamine $D_2$ und $D_3$ besteht in der Stabilisierung des Plasma-$Ca^{++}$ - und Plasma-Phosphat-Spiegels; sie wirken einem Absinken des Plasma-$Ca^{++}$ -Spiegels entgegen.

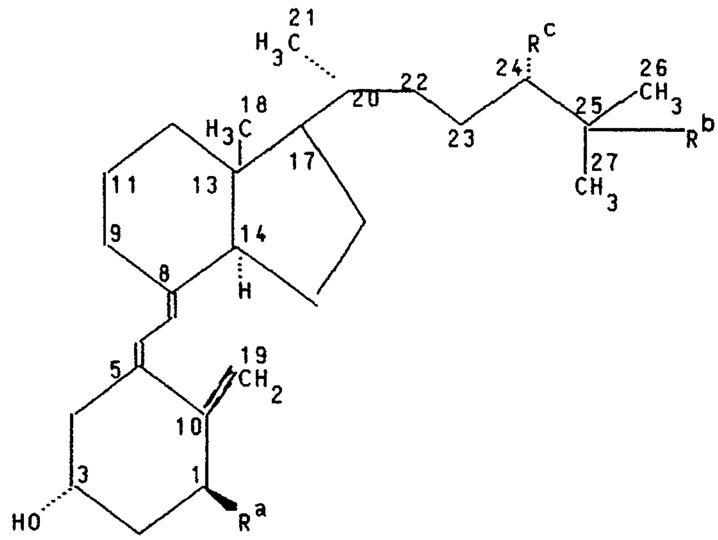

(A)

Ergocalciferol: $R^a = R^b = H$, $R^c = CH_3$, Vitamin $D_2$
Doppelbindung C-22/23
Cholecalciferol: $R^a = R^b = R^c = H$ Vitamin $D_3$
25-Hydroxycholecalciferol: $R^a = R^c = H$, $R^b = OH$
1α-Hydroxycholecalciferol: $R^a = OH$, $R^b = R^c = H$
1α,25-Dihydroxycholecalciferol: $R^a = R^b = OH$, $R^c = H$ Calcitriol

Außer ihrer ausgeprägten Wirkung auf den Calcium- und Phosphatstoffwechsel besitzen Vitamin $D_2$ und $D_3$ und seine synthetischen Abkömmlinge auch proliferationshemmende und zelldifferenzierende Wirkungen (H.F. De Luca, The Metabolism and Function of Vitamin D in Biochemistry of Steroid Hormones, Hrsg. H.L.J. Makin, 2nd Edition, Blackwell Scientific Publications 1984, S. 71-116). Bei Vitamin D-Anwendung kann es aber zu Überdosierungserscheinungen kommen (Hypercalcämie).

Neben den erfindungsgemäßen Verbindungen gibt es bereits eine Reihe von Oxaderivaten in der Vitamin D-Reihe. Beschrieben sind 20-Oxa-, 22-Oxa- und 23-Oxa-Analoga des Calcitriols (23-Oxa; US-Patent 4 772 433, Erf. R. Hesse, 1988; 22-Oxa: E. Murayama et al., Chem. Pharm. Bull. 34,4410, 1987; 20-Oxa: J. Abe et al., FEBS Lett. 222, 58, 1987). Ein 24-Oxa-Analoges des Calcitriols ist chemisch nicht vorstellbar, da es gemäß seiner Natur als offenkettiges Hemiacetal in wäßrigem Milieu sofort zerfallen würde.

Wie DeLuca et al. (Proc. Natl. Acad. Sci. USA 84, 2610, 1987) zeigen konnten, weisen auch 24-Homo-Derivate des Calcitriols noch hohe Affinität zum Calcitriol-Rezeptor auf.

Ein Teil der erfindungsgemäßen Verbindungen, die als 24-Oxa-24-Homo-Analoga des Calcitriols klassifiziert werden müssen, stellen somit eine neuartige Kombination von bekannten wirksamkeitssteigernden Strukturmerkmalen dar.

Es wurde nun gefunden, daß die erfindungsgemäßen 24-Oxa Vitamin-D-Derivate der allgemeinen Formel I sich durch hohe Affinität zum Calcitriol-Rezeptor auszeichnen und auch in hoher Dosierung keine Anstiege des Calciumspiegels im Plasma bewirken. Die proliferationshemmenden Eigenschaften des Calcitriols bleiben jedoch unvermindert erhalten (Dissoziation).

Die Vitamin-D-Aktivität der erfindungsgemäßen Verbindungen wird mittels des Calcitriol-Rezeptortests bestimmt. Er wird unter Verwendung eines spezifischen Rezeptorproteins aus dem Darm rachitischer Hühner durchgeführt. Rezeptorhaltiges Bindungsprotein wird mit $^3$H-Calcitriol (0,5 ng/ml) in einem Reaktionsvolumen von 0,575 ml in Abwesenheit und in Anwesenheit der Prüfsubstanzen für eine Stunde in einem Teströhrchen inkubiert. Zur Trennung von freiem und rezeptorgebundenem Calcitriol wird eine Charcoal-Dextran-Absorption durch geführt. Dazu werden 200 µl einer Charcoal-Dextran-Suspension jedem Teströhrchen zugeführt und bei 22 °C für 30 Minuten inkubiert. Anschließend werden die Proben bei 1500 x g 10 Minuten bei 4 °C zentrifugiert. Der Überstand wird dekantiert und nach ca. 1stündiger Äquilibrierung in Atom-Light in einem β-Zähler gemessen.

Die mit verschiedenen Konzentrationen der Prüfsubstanz sowie der Referenzsubstanz (unmarkiertes Calcitriol) für die Verdrängung von $^3$H-markierter Referenzsubsstanz ($^3$H-Calcitriol) erhaltenen Kompetitionskurven werden in Beziehung zueinander gesetzt und ein Kompetitionsfaktor (KF) ermittelt.

Er ist definiert als Ouotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Demnach besitzt
1α,25-Dihydroxy-26,27-dimethyl-24-oxa-24-homo-cholecalciferol einen KF-Wert von 3 und 1α,25-Dihydroxy-24-oxa-24-homo-cholecalciferol einen KF-Wert von 7.

Durch das stark verminderte Hypercalciämie-Risiko eignen sich die erfindungsgemäßen Substanzen in besonderer Weise zur Herstellung von Arzneimitteln für die Behandlung von Erkrankungen, die durch eine Hyperproliferation gekennzeichnet sind, z.B. hyperproliferative Erkrankungen der Haut (Psoriasis) und maligne Tumoren (Leukämie, Coloncarcinom, Mammacarcinom). In einer besonders bevorzugten Ausführungsform der Erfindung werden vor der Behandslung im Zielorgan Calcitriolrezeptoren nachgewiesen.

Die vorliegende Erfindung bezieht sich somit auch auf pharmazeutische Präparate, die mindestens eine Verbindung gemäß der allgemeinen Formel I zusammen mit einem pharmazeutisch verträglichen Träger enthalten.

Die Verbindungen können formuliert werden als Lösungen in pharmazeutisch verträglichen Solventien oder als Emulsionen, Suspensionen oder Dispersionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch eine andere pharmazeutisch verträgliche und nichttoxische Hilfsstoffe enthalten, wie z.B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch in Form von Cremes, Salben, Lotions oder geeigneter transdermaler Pflaster appliziert, wie in der EP-A-0387 077 beschrieben ist.

Die tägliche Dosis liegt bei
0,1 μg/Patient/Tag - 1000 μg/Patient/Tag,
vorzugsweise
1,0 μg/Patient/Tag - 500 μg/Patient/Tag.

Die erfindungsgemäßen Verbindungen werden im allgemeinen verabreicht analog zur Verabreichung des bekannten Mittels "Calcipotriol" zur Behandlung der Psoriasis

Außerdem betrifft die Erfindung die Verwendung der Verbindungen gemäß Formel I zur Herstellung von Arzneimitteln.

Die Herstellung der 24-Oxa-Vitamin D-Derivate der Formel I erfolgt erfindungsgemäß dadurch, daß eine Verbindung der allgemeinen Formel II

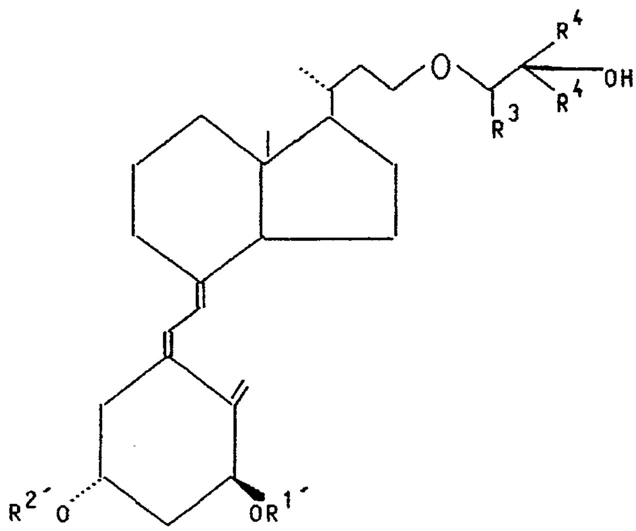

(II),

worin

$R^{1'}$ und $R^{2'}$ für Hydroxyschutzgruppen stehen sowie $R^3$ und $R^4$ die in Formel I angegebene Bedeutung haben, durch Abspaltung dieser Hydroxyschutzgruppen in die freie Trihydroxyverbindung (Verbindung der allgemeinen Formel I, worin $R^1 = R^2 = -R^5 = H$) und gewünschtenfalls diese durch teilweise oder vollständige Veresterung der freien Hydroxygruppen in die entsprechende Acylverbindung (Verbindung der allgemeinen Formel I, worin $R^1$ und/oder $R^2$ und/oder $R^5$ eine $C_1$ -$C_9$ -Acylgruppe bedeutet(n)) überführt wird.

Als Hydroxyschutzgruppen $R^{1'}$ und $R^{2'}$ kommen in erster Linie tertiäre Silylgruppen, beispielsweise der Trimethylsilyl- oder der tert. -Butyl-dimethylsilyl-Rest, infrage. Deren Abspaltung gelingt z.B. unter Verwendung von Tetra-n-butyl-ammoniumfluorid.

Nach der Schutzgruppenabspaltung können freie Hydroxygruppen gewünschtenfalls verestert werden. Die Veresterung der verschiedenen freien Hydroxygruppen kann nach gängigen Verfahren partiell oder vollständig mit dem entsprechenden Carbonsäurehalogenid (Halogenid = Chlorid, Bromid) oder Carbonsäureanhydrid erfolgen.

Es ist auch möglich, eine tertiäre 25-Hydroxygruppe bereits vor der Schutzgruppenabspaltung oder vor der Photoisomerisierung zu verestern.

Als Ausgangsmaterial für die Herstellung der erfindungsgemäß zu verwendenden Ausgangsverbindungen der allgemeinen Formel II dienen Alkohole der allgemeinen Formel III

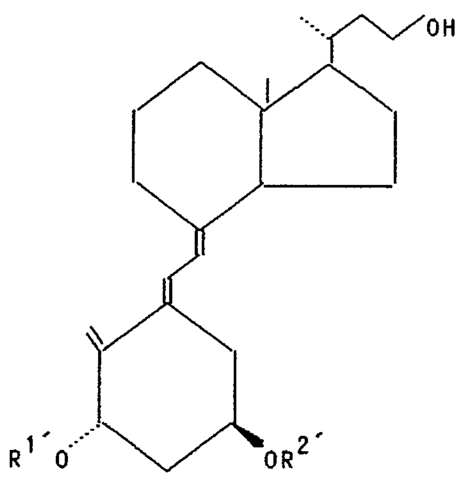

(III),

worin

$R^{1'}$ und $R^{2'}$ die bereits angegebene Bedeutung haben.

1S,3R-Bis-(tert.-Butyldimethylsilyloxy)-24-nor-9,10-secochola-5E,7E,10(19)-trien-23-ol kann z.B. nach US Patent 4,512,925 (Erf.: DeLuca et al., 1985) hergestellt werden.

Durch Umsetzung einer Verbindung der allgemeinen Formel III mit Bromessigsäuretert. -butylester im Zweiphasensystem Toluol/25% Natronlauge in Gegenwart eines Phasentransferkatalysators (im Rahmen vorliegender Erfindung wird Tetra-n-butyl-ammoniumhydrogensulfat oder -fluorid verwendet) gelangt man zu den Estern der Formel IV

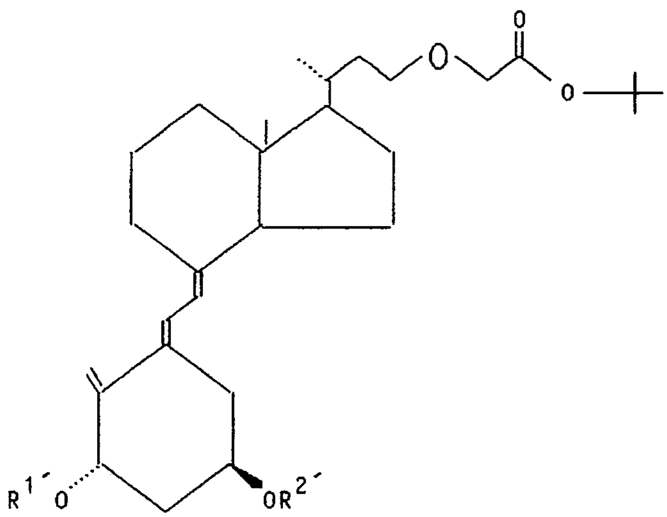

(IV),

in hoher Ausbeute.

Werden Endprodukte der allgemeinen Formel I gewünscht in denen $R^3$ eine Alkylgruppe mit 1 bis 4 Kohlenstoffatomen bedeutet, so wird zunächst die Zwischenstufe IV in Gegenwart einer starken Base wie etwa Lithiumdiisopropylamid in einem aprotischen Lösungsmittel wie Tetrahydrofuran mit einem Alkylhalogenid des allgemeinen Typs $R^3$ Hal ($R^3$ $= C_1$ $-C_4$ -Alkyl, Hal= Br, I) an der der Carbonylgruppe benachbarten Methylengruppe alkyliert.

Die so gewonnenen Verbindungen der allgemeinen Formel V

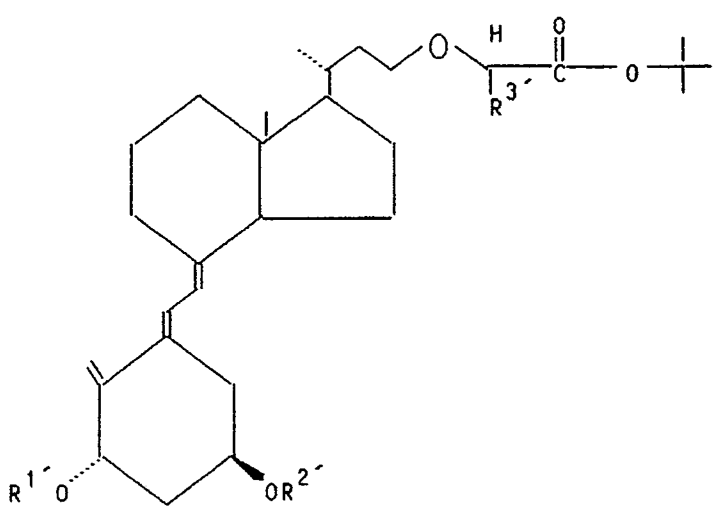

(V),

werden im allgemeinen als Diastereomerengemische erhalten und ohne Auftrennung in die Folgereaktionen eingesetzt.

Diese Zwischenstufe IV läßt sich mit Grignardreagenzien der allgemeinen Formel $R^4$ MgX ($R^4$ $= C_1$ $-C_4$ -Alkyl, X=C1, Br, I) in einem Überschuß von 2 bis 10 Molequivalenten in die Zwischenprodukte der allgemeinen Formel VI

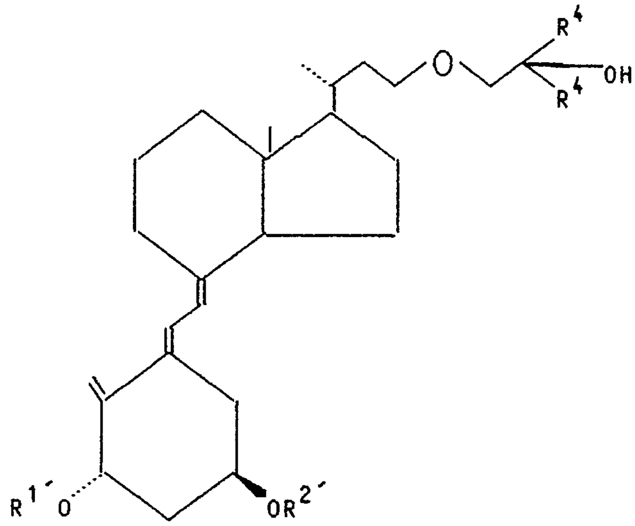

(VI),

überführen.

Die Reduktion der Estergruppe in den Verbindungen der Formel IV oder der Formel V führt zu den Verbindungen der allgemeinen Formel VII

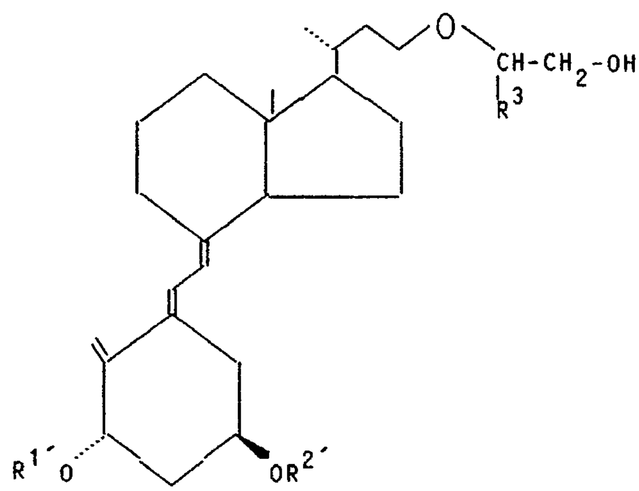

(VII),

worin

$R^3$ ein Wasserstoffatom oder eine lineare oder verzweigte $C_1$ -$C_4$ -Alkylgruppe bedeutet und $R^{1'}$ und $R^{2'}$ die bereits angegebene Bedeutung haben.

Nach Standardverfahren der Vitamin D-Chemie durch Bestrahlung mit ultraviolettem Licht in Gegenwart eines sogenannten "Triplettsensibilisators" (im Rahmen vorliegender Erfindung wird hierfür Anthracen verwendet) lassen sich anschliessend die Verbindungen der allgemeinen Formeln VI und VII in die Verbindungen der allgemeinen Formel II überführen. Durch Spaltung der pi-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt.

Schlüsselschritt zur Synthese der Ausgangsverbindungen der allgemeinen Formel II ist die Umsetzung des Alkohols II mit Bromessigsäure-tert.-butylester unter Phasentransferbedingungen. Dem Fachmann ist bekannt, daß entsprechende Umsetzungen mit Bromessigsäure-methyl(ethyl)-ester äußerst unbefriedigend verlaufen, da es im wesentlichen nur zur Umesterung und nicht zur Bildung der gewünschten Veretherungsprodukte kommt.

Überraschend ist weiterhin die Tatsache, daß der tert.-Butylester IV sich problemlos mit Alkyl-Grignardreagenzien umsetzen läßt, obwohl die sterische Hinderung durch die tert. -Butylgruppe eine erheblich verminderte Reaktivität hätte erwarten lassen.

Die nachfolgenden Beispiele dienen der näheren Erläuterung der Erfindung:

**BEISPIEL 1**

**1α,25-Dihydroxy-26,27-dimethyl-24-oxa-24-homo-cholecalciferol**

a) Eine Lösung von 1S,3R-Bis-(tert.-butyldimethylsilyloxy)-24-nor-9,10-secochola-5E,7E,10(19)-trien-23-ol in 14 ml Toluol wird nach Zugabe von 1,49 ml Bromessigsäure-tert-butylester und 5,5 ml 25%iger Natronlauge und 26,6 mg Tetra-n-butylammoniumhydrogensulfat 24 Stunden bei Raumtemperatur gerührt. Anschließend gibt man erneut 30 mg Tetrabutyl-ammoniumsalz hinzu und rührt weitere 24 Stunden bei 25° C. Zur Aufarbeitung verdünnt man mit Diethylether, wäscht mit Wasser und gesättigter Kochsalzlösung, trocknet die Etherphase über $Na_2SO_4$ und engt ein. Das Rohprodukt wird an 160 g Kieselgel mit Hexan/ Ethylacetat (0-5%) gradientenchromatographiert. Man erhält 1,15 g 1S,3R-Bis-(tert.-butyldimethylsilyloxy)-25-(tert.-butylcarbonyloxy)-24-oxa-26,27-dinor-9,10-secocholesta-5E,7E,10(19)-trien als farbloses Öl.

b) Aus 297 mg Magnesium (Späne) und 0,91 ml Bromethan in 7 ml Tetrahydrofuran stellt man in üblicher Weise Ethylmagnesiumbromid her und tropft bei Raumtemperatur eine Lösung von 750 mg des unter a) erhaltenen Oxa-esters in 10 ml abs. THF hinzu. Nach Zugabe rührt man 1,5 Stunden bei 25° C, gießt in Wasser und extrahiert mit Ethylacetat. Chromatographie des Rohprodukts an Kieselgel mit Hexan/Ethylacetat ergibt 520 mg 1S,3R-Bis-(tert.-butyldimethylsilyloxy)-26,27-dimethyl-24-oxa-24-homo-9, 10-secocholesta-5E,7E,10(19)-trien -25-ol als farbloses Öl.

$^1$H-NMR ($CDCl_3$): δ=0,54 ppm(s,3H,H-18); 3,37(AB-qu.,J=10,5Hz,2H,$OCH_2$); 3,48 (m,2H,$CH_2O$); 4,22-(m,1H,H-3); 4,53(m,1H,H-1); 4,93 u. 4,98 (m,je 1H,H-19); 5,82 u. 6,46(d, J=11HZ,je 1H,H-6,H-7).

c) Eine Lösung von 500 mg des unter b) erhaltenen Produkts in 90 ml Toluol wird nach Zusatz von 88 mg Anthracen und 0,05 ml Triethylamin 18 Minuten bei Raumtemperatur in einer Tauchapparatur (Pyrex-Glas) mit einer Quecksilberhochdrucklampe (Heraeus TQ 150) bestrahlt. Nach dem Einengen der Reaktionslösung wird der Rückstand an Kieselgel mit Hexan/Ethylacetat chromatographiert. Man erhält 490 mg 1S,3R-Bis-(tert.-butyldimethyl-silyloxy)-26,27-dimethyl-24-oxa-24-homo-9, 10-secocholesta-5Z,7E,10(19)-trien -25-ol als farbloses Öl.

d) Eine Lösung von 480 mg des unter c) erhaltenen Produkts in 15 ml THF wird nach Zusatz von 3 ml einer 1molaren Lösung von Tetra-n-butylammoniumfluorid in THF 60 Minuten bei 50° C gerührt. Nach dem Abkühlen verdünnt man mit Ethylacetat, wäscht mit $NaHCO_3$ -Lösung und Wasser, trocknet über $Na_2SO_4$ und engt ein. Chromatographie des Rohprodukts an Kieselgel mit Hexan/Ethylacetat ergibt 200 mg 1α,25-Dihydroxy-26,27-dimethyl-24-oxa-24-homo-cholecalciferol als amorphen Feststoff.

$^1$H-NMR($CDCl_3$) : δ=0,55 ppm(s,3H,H-18); 0,87(t,J=7Hz,$CH_2CH_3$); 0,94(d,J=7Hz,3H, H-21); 1,50-(m,$CH_2CH_3$); 3,26(AB-qu.,J=10,5Hz,2H,$CH_2O$); 3,48(m,2H,$CH_2O$); 4,22 (m,1H,H-3); 4,43(m,1H,H-1); 5,00 u. 5,32(m,je 1H,H-19); 6,02 u. 6,38(d,J= 11Hz, je 1H,H-6,H-7).

**BEISPIEL 2**

**1α,25-Dihydroxy-24-oxa-24-homo-cholecalciferol**

Die Herstellung der Titelverbindung erfolgt in Analogie zu dem unter Beispiel 1 beschriebenen Verfahren. Im Verfahrensschritt 1b) wird lediglich Ethylmagnesiumbromid durch Methylmagnesiumbromid (1,5 molare Lösung in THF/Toluol) ersetzt. Man erhält die Titelverbindung als farbloses Öl.

$^1$H-NMR($CDCl_3$): α=0,53 ppm(s,3H,H-18); 0,95(d,J=7Hz,3H,H-21); 1,20(s,6H,H-26,H-27); 3,23(AB-qu.,J=10,5Hz,2H,$CH_2O$); 3,50(m,2H,$CH_2O$); 4,23(m,1H,H-3); 4,43(m, 1H,H-1); 5,00 u. 5,32(m, je 1H,H-19); 6,02 u. 6,38 (d,J=11Hz, je 1H,H-6,H-7).

**BEISPIEL 3**

**1α,26-Dihydroxy-24-oxa-cholecalciferol**

a) Eine Lösung von 0,6 ml Diisopropylamin in 6 ml THF wird bei 0° C tropfenweise mit 2,54 ml einer 1,6 molaren Lösung von n-Buthyllithium in Hexan versetzt. Man rührt 15 Minuten bei 0° C, kühlt dann auf -70° C und tropft eine Lösung von 1,00 g 1S,3R-Bis-(tert.-butyldimethylsilyloxy)-25-(tert.-butylcarbonyloxy)-24-oxa-26,27-dinor-9, 10-secocholesta-5E,7E,10(19)-trien (s. Beispiel 1a) in 15 ml THF hinzu. Nach Zugabe rührt man 60 Minuten bei -70° C und gibt dann tropfenweise 0,36 ml Jodmethan hinzu. Die Reaktionslösung wird 30 Minuten bei -70° C und weitere 30 Minuten bei Raumtemperatur gerührt, in Wasser gegossen und mit Ethylacetat extrahiert. Chromatographie des Rohprodukts an

Kieselgel mit Hexan/ Ethylacetat ergibt 730 mg 1S,3R-Bis-(tert.-butyldimethylsilyloxy)-25(tert.-butylcarbonyloxy)-24-oxa-27-nor-9,10-secocholesta-5E,7E,10(19)-trien als öliges Gemisch der C-25-Epimeren.

b) Das unter a) erhaltene Produkt (730 mg) wird in 100 ml Toluol, 140 mg Antracen und 0,05 ml Triethylamin unter den Bedingungen des Beispiels 1c) photoisomerisiert. Man erhält 610 mg 1S,3R-Bis-(tert.-butyldimethylsilyloxy)-25-(tert.-butylcarbonyloxy)-24-oxa-27-nor-9, 10-secocholesta-5Z,7E,10(19)-trien als farbloses Öl.

c) 380 mg des unter b) erhaltenen Epimerengemisches werden in 10 ml THF gelöst und bei 5° C tropfenweise zu einer Suspension von 65 mg Lithiumaluminiumhydrid in 10 ml THF gegeben. Man rührt 45 Minuten bei 5° C, zerstört dann überschüssiges Reduktionsmittel durch vorsichtige Zugabe von wäßrigem THF, filtriert und engt das Filtrat ein. Das so erhaltene rohe 1S,3R-Bis-(tert.-butyldimethylsilyloxy)-24-oxa-9, 10-secocholesta5Z,7E,10(19)-trien-26-ol wird unter den Bedingungen des Beispiels 1 d) mit Tetra-n-butylammoniumfluorid desilyliert. Nach chromatographischer Reinigung erhält man 110 mg 1α,26-Dihydroxy-24-oxa-cholecalciferol als öliges Gemisch der C-25 Epimeren im ungefähren Verhältnis 1:1.

$^1$H-NMR($CDCl_3$): δ=0,54 ppm(s,3H,H-18); 0,96(d,J=7Hz,3H,H-21); 1,10(2d,J=6Hz, 3H,H-27); 4,22-(m,1H,H-3); 4,42(m,1H,H-1); 5,00 u. 5,32(s, je 1H,H-19); 6,00 u. 6,38(d,J=11Hz, je1H,H-6,H-7).

## Patentansprüche

1. 24-Oxa-Derivate in der Vitamin D-Reihe der allgemeinen Formel I

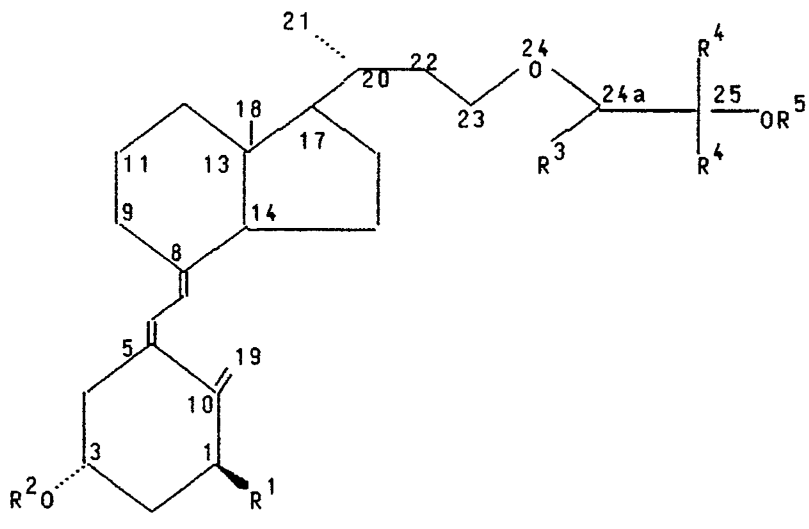

(I),

worin

| | |
|---|---|
| $R^1$, $R^2$ und $R^5$ | unabhängig voneinander ein Wasserstoffatom oder eine Acylgruppe mit 1 bis 9 Kohlenstoffatomen |
| $R^3$ | ein Wasserstoffatom oder eine lineare oder verzweigte Alkylgruppe mit 1 bis 4 Kohlenstoffatomen und |
| $R^4$ | je ein Wasserstoffatom oder je eine lineare oder verzweigte Alkylgruppe mit 1 bis 4 Kohlenstoffatomen |

bedeuten,

2. Verbindungen nach Anspruch 1, dadurch gekennzeichnet, daß $R^1$, $R^2$ und $R^5$ Wasserstoffatome bedeuten.

3. Verbindungen nach Anspruch 1, dadurch gekennzeichnet, daß $R^3$ für ein Wasserstoffatom steht.

4. Verbindungen nach Anspruch 1, dadurch gekennzeichnet, daß $R^3$ für eine Methyl-, Ethyl-, Propyl- oder Isopropylgruppe steht.

5. Verbindungen nach Anspruch 1, dadurch gekennzeichnet, daß $R^4$ je für eine Methyl-, Ethyl-, Propyl- oder Butylgruppe steht.

6. 1α,25-Dihydroxy-24-oxa-24-homo-cholecalciferol,
1α,25-Dihydroxy-26,27-dimethyl-24-oxa-24-homo-cholecalciferol,
26,27-Diethyl-1α,25-Dihydroxy-24-oxa-24-homo-cholecalciferol,
1α,25-Dihydroxy-24-oxa-26,27-di-n-propyl-24-homo-cholecalciferol,
1α,26-Dihydroxy-24-oxa-cholecalciferol,
1α,26-Dihydroxy-27-methyl-24-oxa-cholecalciferol.

7. Verfahren zur Herstellung von 24-Oxa-Derivaten in der Vitamin D-Reihe der allgemeinen Formel I

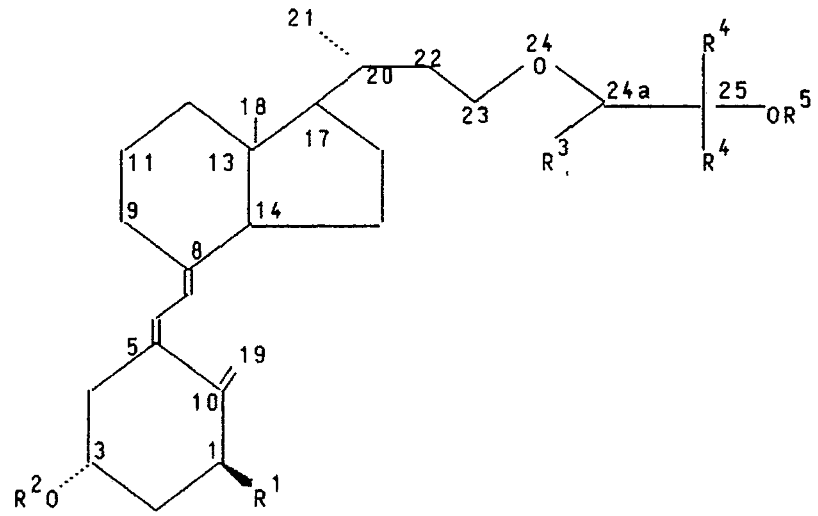
(I),

worin

| | |
|---|---|
| $R^1$, $R^2$ und $R^5$ | unabhängig voneinander ein Wasserstoffatom oder eine Acylgruppe mit 1 bis 9 Kohlenstoffatomen |
| $R^3$ | ein Wasserstoffatom oder eine lineare oder verzweigte Alkylgruppe mit 1 bis 4 Kohlenstoffatomen, |
| $R^4$ | je ein Wasserstoffatom oder je eine lineare oder verzweigte Alkylgruppe mit 1 bis 4 Kohlenstoffatomen und |
| | bedeuten, dadurch gekennzeichnet, daß eine Verbindung der allgemeinen Formel II |

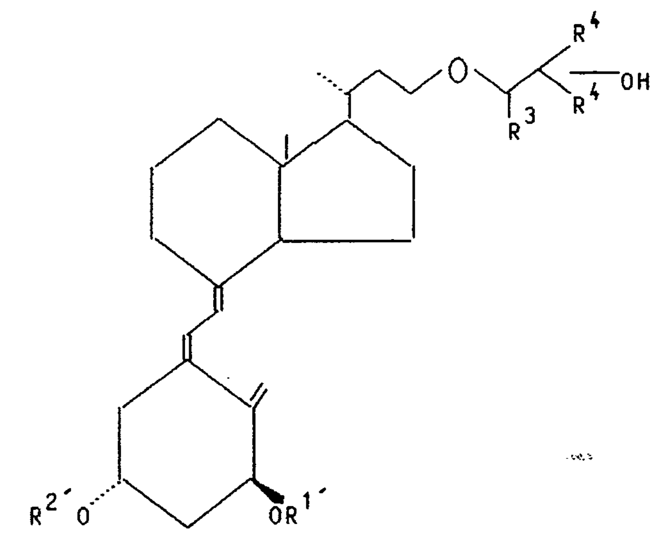
(II),

worin
$R^1$ und $R^2$ für Hydroxyschutzgruppen stehen sowie $R^3$ und $R^4$ die in Formel I angegebene Bedeutung haben, durch Abspaltung dieser Hydroxyschutzgruppen in die freie Trihydroxyverbindung (Verbindung der allgemeinen Formel I, worin $R^1 = R^2 = R^5 = H$) und gewünschtenfalls diese durch teilweise oder vollständige Veresterung der freien Hydroxygruppen in die entsprechende Acylverbindung (Verbindung der allgemeinen Formel I, worin $R^1$ und/oder $R^2$ und/oder $R^5$ eine $C_1$-$C_9$-Acylgruppe bedeutet(n)) überführt wird.

**8.** Pharmazeutische Präparate, dadurch gekennzeichnet, daß sie mindestens eine Verbindung gemäß den Ansprüchen 1 bis 6 sowie einen pharmazeutisch verträglichen Träger enthalten.

**9.** Verwendung der Verbindungen gemäß den Ansprüchen 1 bis 6 zur Herstellung von Arzneimitteln.

Europäisches Patentamt

# EUROPÄISCHER RECHERCHENBERICHT

Nummer der Anmeldung

**EP 91 25 0091**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 184 112 (CHUGAI SEIYAKU K.K.)<br>* Beispiel 17; Seite 9, Verbindung Nr. XIII; Anspruch 8 *<br>– – – | 1,9 | C 07 C 401/00 |
| A | US-A-4 125 544 (J.H. DYGOS)<br>* Beispiel 8; Anspruch 1 *<br>– – – | 1 | |
| A | PATENT ABSTRACTS OF JAPAN Band 10, Nr. 335 (C-384)(2391), 13. November 1986;<br>& JP - A -61140560 (CHUGAI PHARMACEUT. CO. LTD.) 27.06.1986<br>– – – | 1,8 | |
| A,P | WO-A-9 009 991 (LEO PHARMACEUTICAL PRODUCTS)<br>* Anspruch 1; Tabelle 1, Seite 13 *<br>– – – – – | 1 | |

RECHERCHIERTE SACHGEBIETE (Int. Cl.5)

C 07 C 401/00

Der vorliegende Recherchenbericht wurde für alle Patentansprüche erstellt

| Recherchenort | Abschlußdatum der Recherche | Prüfer |
|---|---|---|
| Berlin | 20 Juni 91 | KAPTEYN H G |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument